# EUROPEAN PATENT APPLICATION

(11) **EP 1 384 463 A1**
(43) Date of publication of application: **28.01.2004**
(21) Application number: 03011321.1
(22) Date of filing: 19.05.2003
(51) Int. Cl.: A61H 33/00, F16L 37/53

(54) **Water system for whirlpool baths**

(30) Priority: 27.06.2002 IT PN20020048
(71) Applicant: DOMINO S.p.A., 33097 Spilimbergo, Pordenone (IT)
(72) Inventor: Bertoia, Giuliano, 33090 Arzene, Pordenone (IT)
(74) Representative: Giugni, Valter

(57) **Abstract**

The present patent application refers to a water system for whirlpool baths comprising a water-supply circuit (5), provided to supply water under pressure to a plurality of water-jet outlets (2, 3), and means for interconnecting said water-jet outlets (2, 3) with an electric pump (6) adapted to supply the water-jet outlets themselves. Said interconnecting means comprise a plurality of conduits (8, 9) that are separate and distinct from each other, in which each such conduit is adapted to directly and individually supply a respective water-jet outlet.

## Description

The present invention refers to a water system for whirlpool baths, in particular such a system for delivering water under pressure into a correspondingly fitted bath tub or pool.

Water systems for whirlpool baths, as they are currently known in the art, generally comprise an electric pump from which there extends a main delivery conduit, usually consisting of a rigid plastic pipe, in which water under pressure is circulated to supply the various water-jet outlets provided in the side walls of the bath tub or pool; each one of these water-jet outlets is interconnected, in a firmly joined manner and in series, to the main delivery conduit via a respective offtake conduit that is preferably constituted by T-shaped pipe members interposed in the main delivery conduit in correspondence of each single water-jet outlet.

Some of the drawbacks that are encountered in current, i.e. prior-art water systems for whirlpool baths are brought about exactly by the relative rigidity of the main water delivery pipe, which first of all implies a need for a whole set of sleeves and T-shaped or elbow fittings to be used in order to both reach each water-jet outlet and follow the contour of the tub, thereby determining a resulting interruption in the continuity of the main water delivery pipe and, as a consequence, an unavoidable pressure loss along the same pipe; in addition, the assembly and the shaping of the various conduits and related fittings make the whole water system rather complex to manufacture according to any acceptable industrial engineering standards, further to requiring the use of special adhesives that quite often prove detrimental to both the health and the environment.

Another typical drawback is given by a lack of uniformity in the distribution of the water and/or - where provided - the disinfectant (which is generally let into the water system and the tub at the end of the actual operating cycle via an appropriate supply system) between the first and the last water-jet outlets supplied from the main water delivery pipe: such a lack of uniformity in the distribution of both water and disinfectant, if any, is due to a gradual reduction in the flow rate along the pipe: in fact, the water-jet outlet that is closer to the electric pump will receive an amount of water which is certainly greater than the one received by the water-jet outlet that lies more distant from the pump. In order to compensate for such a drawback, the need arises for the water system to be designed and made in such a manner as to make sure that the pipes and conduits are given an adequate gradient, thereby adding to the complexity in the construction and manufacture of the system.

A further drawback lies in the fact that water may be stagnating in the conduits owing to the imperfect, i.e. poor inner continuity of the main water delivery pipe that is determined by the contiguity of various component parts, such as pipes and fittings, connected to each other.

It therefore is a main object of the present invention to do away with the various drawbacks of prior-art solutions by providing a water system for whirlpool baths which is highly efficient as far as both the process of letting water under pressure into the tub and the subsequent emptying process are concerned.

Within the above general object, it is a purpose of the present invention to provide a water system for whirlpool baths, which is capable of evenly supply water at substantially the same pressure to each one of the water-jet outlets of the tub.

Another purpose of the present invention is to provide a water system for whirlpool baths, which is effective in minimizing the possibility for water to stagnate in the pipes, so as to achieve an improvement in the hygiene and the cleanliness of the water system itself.

A further purpose of the present invention is to provide a water system for whirlpool baths, which has a reduced number of component parts that need to be assembled together, and which therefore is more easily manufactured to more adequate industrial engineering standards.

Still another purpose of the present invention is to provide a water system for whirlpool baths, in which the use of chemical substances, in particular glues or adhesives, is practically eliminated.

A further purpose yet of the present invention is to provide a water system for whirlpool baths, in which the compliance, i.e. adaptation of pipes and conduits to the shape and contour of the tub is more easy to achieve than this is the case with prior-art water systems.

An equally important purpose of the present invention is to provide a water system for whirlpool baths, which is capable of being manufactured at competitive costs with the use of readily available materials, techniques, machinery and tools.

According to the present invention, these aims, along with further ones that will be apparent in the following description, are reached in a water system for whirlpool baths incorporating the characteristics as recited in Claim 1 appended hereto.

Anyway, features and advantages of the water system for whirlpool baths according to the present invention will be more readily understood from the description of a particular, although not sole embodiment that is given below by way of non-limiting example with reference to the accompanying drawings, in which:

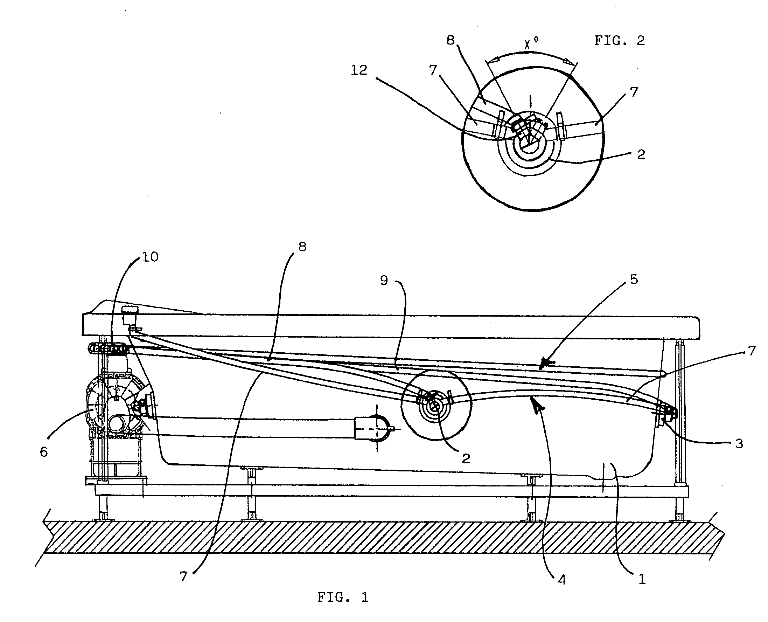

- Figure 1 is a side view of the water system for whirlpool baths according to the present invention;
- Figure 2 is a view of a detail of the water system for whirlpool baths illustrated in Figure 1;

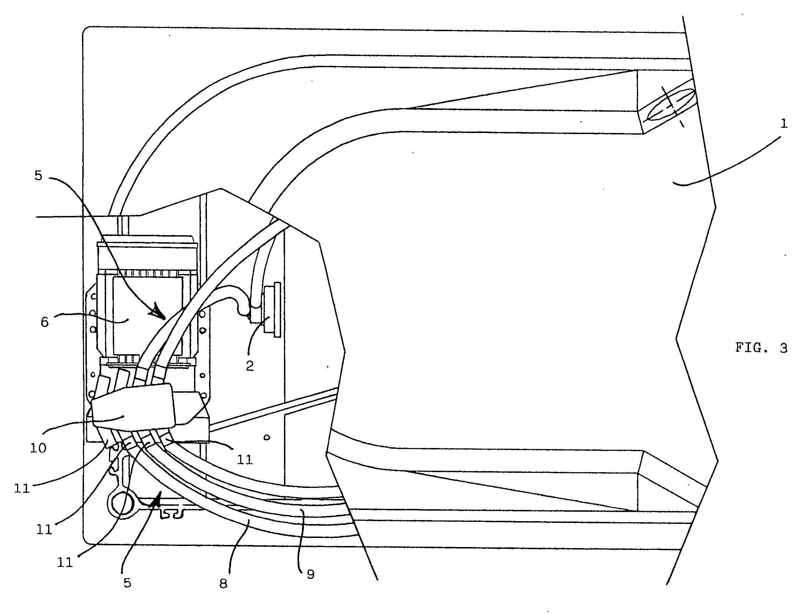

- Figure 3 is a partially cutaway, plan top view of a portion of the water system for whirlpool baths illustrated in Figure 1.

With reference to the above cited Figures, the reference numeral 1 is used to generally indicate a bath-tub provided with a plurality of water-jet outlets 2, 3 arranged along the walls of the tub 1 and communicating with the interior of the same tub 1. These water-jet outlets 2, 3 are supplied with a mixture of air and water under pressure delivered by a water system for whirlpool baths that comprises a pneumatic circuit 4 for introducing air in said water-jet outlets 2, 3, a water-carrying circuit 5 for introducing water in the same water-jet outlets, and an electric pump 6. The pneumatic circuit 4 is substantially constituted by a series of tubes 7 interconnecting said water-jet outlets 2, 3.

According to an innovatory feature of the present invention, the water-carrying circuit 5 is comprised of a plurality of conduits 8, 9, which are advantageously made of a flexible material and directly interconnect the electric pump 6 with each one of said water-jet outlets 2, 3; in particular, each such conduit 8, 9 has an end thereof connected to the electric pump via a distributor 10 with several outlets 11, which is in turn connected to the delivery side of the electric pump 6, whereas the opposite end of said each such conduit is directly connected to the inlet of the respective water-jet outlet 2, 3. A substantially parallel connection is thereby provided between the electric pump 6 and the water-jet outlets 2, 3.

In an advantageous manner, said distributor 10 is mounted on the electric pump 6 at a higher level with respect to the water-jet outlets 2, 3, relative to the resting plane of the tub 1, such that the conduits 8, 9 are able to extend along the periphery of the tub 1 with a slight sloping gradient in the direction of the water-jet outlets 2, 3. Furthermore, the outlets 11 of the distributor 10 are preferably all positioned on the same plane in such a manner as to be able to supply all water-jet outlets 2, 3 in a substantially well-balanced and even manner.

In order to facilitate the connection of the inlet of the water-jet outlets 2, 3 with the respective conduits 8, 9, the attachment pipe 12 provided to connect an end of each such conduit 8, 9 to the respective water-jet outlet 2, 3 can be advantageously mounted pivotally on the same water-jet outlet 2, 3; in this manner, the conduits 8, 9 are not forced into taking any markedly curved configurations and are on the contrary capable of arranging themselves in the most congenial manner to the benefit of the regularity of the water flow circulating in the same conduits.

At the end of the operating cycle of the water system, inside the water-carrying circuit 5 itself there may be circulated - wherever such a feature is also provided - a liquid disinfectant aimed at ensuring the hygiene and cleanliness of the tub 1: the afore cited direct supply will in this case enable the water-jet outlets 2, 3 to receive such a disinfectant in a substantially uniform and well-balanced manner, thereby clearly improving the sanitation and cleansing effect.

Fully apparent from the above description is therefore the ability of the water system for whirlpool baths according to the present invention to effectively reach the afore cited aims and advantages: in fact, the direct connection, preferably via flexible conduits, between the electric pump 6 and each water-jet outlet 2, 3 of the tub 1 enables all such water-jet outlets 2, 3 to be supplied in a most uniform manner at substantially the same pressure of the water; it also enables the water to be drained, i.e. let off from the same water-jet outlets in an equally uniform and substantially complete manner at the end of the operating cycle of the system, thereby ensuring a high efficiency both in the process of letting water under pressure into the tub and the subsequent emptying process.

It can therefore be readily appreciated how the water system for whirlpool baths according to the present invention is to a far lesser extent likely to enable water to stagnate in the system, while ensuring - wherever such a feature is provided - an optimum disinfecting effect thanks to the afore described direct supply of the water-jet outlets and the complete elimination of offtake conduits, thereby clearly improving the hygiene and the cleanliness of the same water system in general.

Furthermore, such a direct supply of the water-jet outlets enables the various component parts that need to be assembled together to be eliminated or, at least, the number thereof to be drastically reduced, thereby introducing a greater simplicity in the manufacturing process and doing practically away with the use of chemicals that may prove detrimental to both the health and the environment.

The reduction in the number of component parts implies also a reduction in the number of connections to be made with the use of sleeve couplings, T-shaped or elbow fittings, and the like, thereby determining a resulting greater regularity in the flow of the water and, therefore, smaller pressure losses along the conduits.

Finally, the possible use of flexible conduits allows for the same conduits to be much more easily adapted to the actual shape and contour of the bath-tub, thereby reducing the time needed to complete the finished product.

It will be appreciated that the afore described water system for whirlpool baths may be subject to a number of different applications, modifications and variants without departing from the scope of the present invention.

It should be finally noticed that the materials used to manufacture the water system for whirlpool baths of the present invention, as well as the shapes and the sizing of the individual component parts thereof, may each time be selected so as to more appropriately meet the particular requirements or suit the particular application, again without departing from the scope of the present invention.

## Claims

1. Water system for whirlpool baths comprising a water-carrying circuit (5) adapted to supply water under pressure to a plurality of water-jet outlets (2, 3), interconnection means (8, 9) being provided between said water-jet outlets (2, 3) and at least an electric pump (6) adapted to supply said water-jet outlets (2, 3), **characterized in that** said interconnection means comprise a plurality of conduits (8, 9) that are separate and distinct from each other, in which each such conduit (8, 9) is adapted to directly and individually supply a respective one of said plurality of water-jet outlets (2, 3).

2. Water system according to claim 1, in which said conduits (8, 9) are made of a flexible material.

3. Water system according to claim 1, in which each one of said conduits (8, 9) is connected at an end thereof to the delivery side of said electric pump (6) under the interposition of a distributor (10) provided with several outlets (11).

4. Water system according to claim 3, in which said outlets (11) are positioned on the same horizontal plane.

5. Water system according to claim 3, in which each one of said conduits (8, 9) is directly connected at the opposite end thereof to the inlet of the respective water-jet outlet (2, 3).

6. Water system according to any of the preceding claims or a combination thereof, in which each one of said water-jet outlets (2, 3) is provided with an attachment pipe (12) adapted to connect said water-jet outlet with the respective conduit (8, 9), said attachment pipe (12) being pivotally mounted on said water-jet outlet.

7. Water system according to any of the preceding claims or a combination thereof and further comprising a dispenser of liquid disinfectant, in which said plurality of conduits (8, 9) is fluidly connected with said liquid disinfectant dispenser.

8. Water system for whirlpool baths, **characterized by** what has been described and/or illustrated in and with reference to the accompanying drawings.
